# NOUVEAU FASCICULE DE BREVET EUROPEEN

(11) **EP 1 413 248 B2**
(45) Date de publication et mention de la décision concernant l'opposition: **23.09.2009**
(45) Mention de la délivrance du brevet: 26.07.2006
(21) Numéro de dépôt: 03292830.1
(22) Date de dépôt: 28.02.2002
(51) Int. Cl.: A61B 5/103

(54) **Evaluation du relief de la peau**
Hautreliefsauswertung
evaluation of the skin relief

(30) Priorité: 02.03.2001 FR 0102888
(43) Date de publication de la demande: 28.04.2004
(62) Demande divisionnaire de: 02706910.3
(73) Titulaire: L'ORÉAL, 75008 Paris (FR)
(72) Inventeur: Garnier, Pierre, 75003 Paris (FR)
(74) Mandataire: Tanty, François

(56) Documents cités:
- FR-A- 2 063 743
- US-A- 5 684 573

## Description

La présente invention concerne un procédé d'évaluation du vieillissement de la peau.

Il est connu, pour visualiser les signes de vieillissement de la peau, de réaliser une empreinte de cette dernière au moyen d'une matrice malléable siliconée, par exemple celle commercialisée sous la dénomination commerciale *Silflow.* Un tel procédé est assez délicat et coûteux à mettre en oeuvre, car il est nécessaire d'utiliser un appareillage de mesure relativement complexe, ce qui impose la présence d'un personnel spécialement formé et entraîné à la prise d'empreinte. Ce procédé est inadapté à une mise en oeuvre par les consommateurs eux-mêmes ou en ambulatoire, par exemple sur un point de vente.

Il est connu, par ailleurs, de déterminer le degré de sécheresse de la peau en prélevant des cornéocytes au niveau du stratum cornéum au moyen d'un support adhésif tel que décrit dans le brevet US 5 088 502 et commercialisé par la société *Cuderm Corporation* sous la marque déposée *D-Squame*.

Le brevet US 5 684 573 propose un dispositif perfectionné d'analyse de la peau, en laboratoire.

Le brevet FR 2 063 743 décrit un support permettant de déterminer par impression le type de la peau de l'utilisateur, le support comportant une surface adhésive. Ce support peut être utilisé pour la prescription, la vente et la publicité de produits de beauté, en particulier la vente par correspondance.

L'article "A simple method for the study of scale pattern and effects of a moisturizer-qualitative and quantitative evaluation by D-Squame tape compared with parameters of epidermal hydration" Clinical and Experimental Dermatology 1989; 14 : 277-282 divulgue l'utilisation de D-Squame pour évaluer l'hydratation.

Il existe un besoin pour évaluer l'état de vieillissement de la peau, d'une manière simple et peu coûteuse, mais suffisamment précise néanmoins.

La présente invention répond à ce besoin, grâce à un procédé tel que defini dans la revendication 1 et un kit tel que defini dans la revendication 17.

La société déposante a constaté que, de manière surprenante, l'utilisation d'un support comportant une surface adhésive, tel que décrit par exemple dans le brevet US 5 088 502, permet de visualiser le relief et notamment les signes de vieillissement, en particulier les ridules et rides présentes à la surface de la peau, en réalisant une empreinte bi-dimensionnelle de la surface de la peau. Le support adhésif, en ne collant qu'aux "plateaux" de la peau, agit à la façon d'un tampon encreur et permet de restituer l'état de surface comme un négatif. La modification de l'aspect de la surface adhésive peut résulter de la présence à sa surface de particules arrachées à la peau, par exemple des cellules mortes ou autres impuretés, et peut résulter aussi du dépôt de particules d'adhésif sur la peau aux endroits où la surface adhésive a adhéré à la peau.

Le support adhésif utilisé peut être transparent. L'examen du support adhésif peut alors s'effectuer en le disposant devant un fond de couleur foncée. Pour une bonne visualisation de l'image, le support adhésif est de préférence déposé sur le fond de couleur foncée sans le faire adhérer à ce dernier.

Le support comporte avantageusement une languette de préhension débordant d'un côté au moins de la surface adhésive.

La zone de test peut être choisie parmi les régions suivantes :
- patte d'oie,
- front,
- coin de la bouche,
cette liste n'étant pas limitative.

Dans l'invention, on compare l'image formée sur la surface adhésive avec des images de référence correspondant à divers degrés de vieillissement de la peau, afin d'en déduire le degré de vieillissement de la peau de la personne ayant subi le test.

Ces images de référence peuvent être imprimées. En variante, ces images de référence peuvent être affichées à l'écran d'un ordinateur.

La comparaison entre l'image formée sur le support et les images de référence peut être effectuée à l'oeil nu. En variante, ou additionnellement, la comparaison entre l'image formée sur le support et les images de référence peut être effectuée de manière automatisée.

L'image formée sur la surface adhésive du support peut être analysée à distance. L'image peut notamment être numérisée avant d'être analysée à distance, afin d'être envoyée sous la forme d'un fichier, par exemple.

Il est possible d'effectuer un traitement de l'image formée sur la surface adhésive en vue de déterminer des paramètres caractéristiques de la zone de test. Un tel traitement peut comporter un comptage des rides ou ridules, une mesure de leurs dimensions et de leur orientation.

Dans une mise en oeuvre de l'invention, on enregistre les images formées sur différents supports appliqués successivement sur la zone de test. Ces images enregistrées peuvent être comparées ensuite afin, par exemple, de mettre en évidence l'effet d'un traitement ou le besoin d'un traitement.

Les images enregistrées peuvent être affichées simultanément pour permettre à une personne de percevoir les effets d'un traitement ou de prendre conscience du besoin d'un traitement.

Le brevet décrit encore pour objet un atlas permettant d'évaluer le relief de la peau, notamment son degré de vieillissement, comportant une pluralité d'images de référence représentative chacune de l'image formée sur un support comportant une zone adhésive, après application sur une zone de test de la peau, ces images présentant des motifs, notamment des lignes ou des points traduisant la présence de rides, de ridules ou de pores sur la zone de test et correspondant par exemple à différents degrés de vieillissement de la peau.

D'autres caractéristiques et avantages de la présente invention ressortiront à la lecture de la description détaillée qui va suivre, d'exemples de mise en oeuvre non limitatifs de l'invention, et à l'examen du dessin annexé, sur lequel :
- la figure 1 illustre l'application d'un support sur une zone de test située au niveau de la patte d'oie,
- la figure 2 représente le support une fois retiré de la peau et sur le point d'être placé devant un fond sombre permettant de mettre en évidence la modification de l'aspect de la surface adhésive,
- la figure 3 est un schéma en blocs illustrant différentes étapes d'un procédé conforme à un exemple de mise en oeuvre de l'invention,
- la figure 4 représente un atlas permettant de comparer l'image formée sur le support avec des images de référence,
- la figure 5 illustre l'affichage sur l'écran d'un ordinateur d'un atlas permettant de comparer l'image formée sur le support avec des images servant de référence,
- les figures 6 à 8 sont des schémas en blocs illustrant différents procédés conformes à des exemples de mise en oeuvre de l'invention,
- la figure 9 représente un kit comportant un produit antirides, une pluralité de supports adhésifs et un emballage sur lequel est imprimé un atlas, et
- la figure 10 représente un support ayant une surface adhésive de contour circulaire.

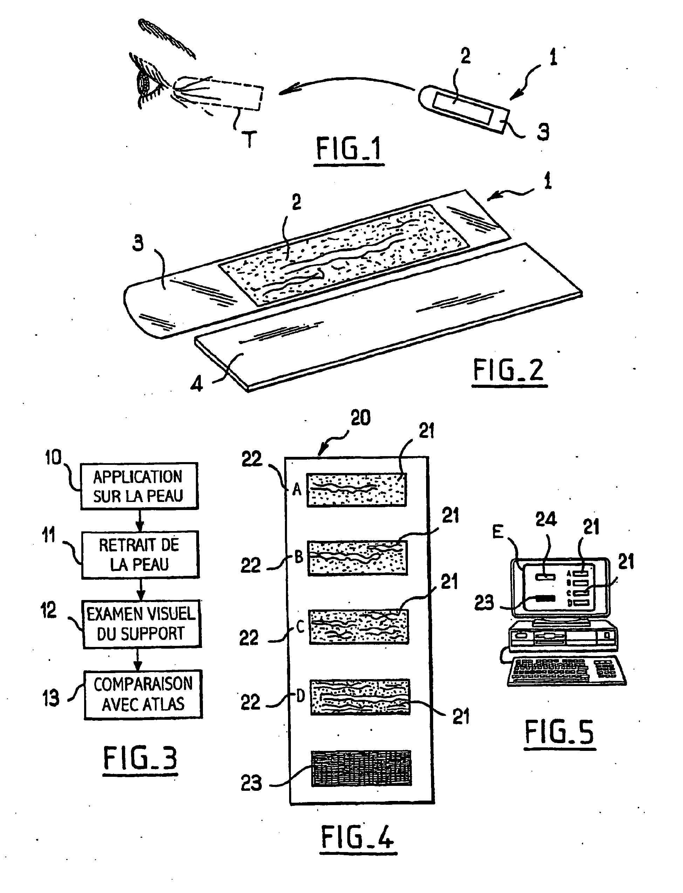

On a représenté sur la figure 1 un support adhésif 1, connu en lui-même, par exemple commercialisé par la société *Cuderm Corporation* sous la marque déposée *D-Squame.*

Ce support comprend une surface adhésive 2 et une languette 3 non adhésive permettant la préhension du support adhésif 1 sans contact des doigts avec la surface adhésive 2. L'adhésif constituant la surface adhésive 2 est par exemple un adhésif solide et peut s'étendre sous la forme d'une couche figée d'épaisseur sensiblement uniforme.

Le support 1 est transparent, dans l'exemple décrit.

Il est revêtu, avant l'utilisation, d'une pellicule de protection amovible, non représentée, couvrant la surface adhésive 2.

Le support 1 est destiné à être appliqué sur la peau, sans qu'une pression trop forte ne soit exercée, sur une zone de test T, située par exemple dans la région de la patte d'oie, comme illustré. La zone de test T a été préalablement démaquillée et nettoyée.

Les particules présentes à la surface de la peau, par exemple des cellules mortes, et situées au niveau des "plateaux" adhèrent à la surface adhésive 2 tandis que les particules situées dans les creux formés entre les "plateaux" par les rides ou ridules ne viennent pas en contact effectif avec la surface adhésive 2, de sorte que lorsque le support 1 est retiré, on obtient sur la surface adhésive 2 une image en négatif faisant apparaître les rides ou ridules présentes dans la zone de test T. Des particules d'adhésif peuvent également rester sur la peau au niveau des « plateaux », ce qui contribue à la modification de l'aspect de la surface adhésive.

Pour mettre en évidence l'image ainsi formée sur la surface adhésive 2, on peut amener le support 1 devant un fond 4 opaque et de couleur foncée, par exemple noir.

Les différentes étapes d'utilisation du support 1 sont résumées dans le schéma en blocs de la figure 3.

On commence par appliquer sur la peau le support 1, ce qui correspond à l'étape 10 de la figure 3.

Ensuite, le support 1 est retiré à l'étape 11 et l'on procède à l'étape 12 à son examen visuel.

Cet examen comporte avantageusement une étape 13 au cours de laquelle l'image formée sur le support est comparée avec des images de référence d'un atlas 20 ou échelle de comparaison, tel que celui représenté à la figure 4.

Les images 21 correspondent à différents degrés de vieillissement de la peau.

Ainsi, l'image 21 du haut correspond par exemple à l'image que l'on obtiendrait sur le support 1 en appliquant celui-ci sur la patte d'oie d'une personne dont la peau est jeune. Les autres images 21 correspondent aux images que l'on obtiendrait après application des supports 1 sur des peaux ayant des degrés de vieillissement de plus en plus importants. De préférence, comme illustré, l'atlas comporte un identifiant alphanumérique en regard de chaque image 21, afin notamment de permettre de repérer l'image 21 retenue, la lettre A correspondant dans l'exemple représenté à l'absence de signes prononcés de vieillissement tandis que la lettre D correspond au degré de vieillissement le plus important, les images 21 repérées par les lettres B et C correspondant à des degrés intermédiaires.

L'atlas 20 peut être réalisé par impression d'un support, par exemple.

En variante, les images peuvent être affichées à l'écran E d'un ordinateur, comme illustré sur la figure 5.

L'atlas 20 peut avantageusement comporter une zone sombre 23, devant laquelle est disposé le support 1 après application sur la peau, de manière à mettre en évidence la modification de l'aspect de la surface adhésive 2.

Lorsqu'un écran E est utilisé, les images 21 peuvent être affichées simultanément avec une zone sombre 23, devant laquelle est disposé le support 1.

En variante, le support 1 peut être positionné en un emplacement prédéterminé 24 de l'écran, au niveau duquel sont successivement affichées des images destinées à permettre à l'observateur de déterminer, en observant l'écran au travers du support 1, le degré de vieillissement de la peau.

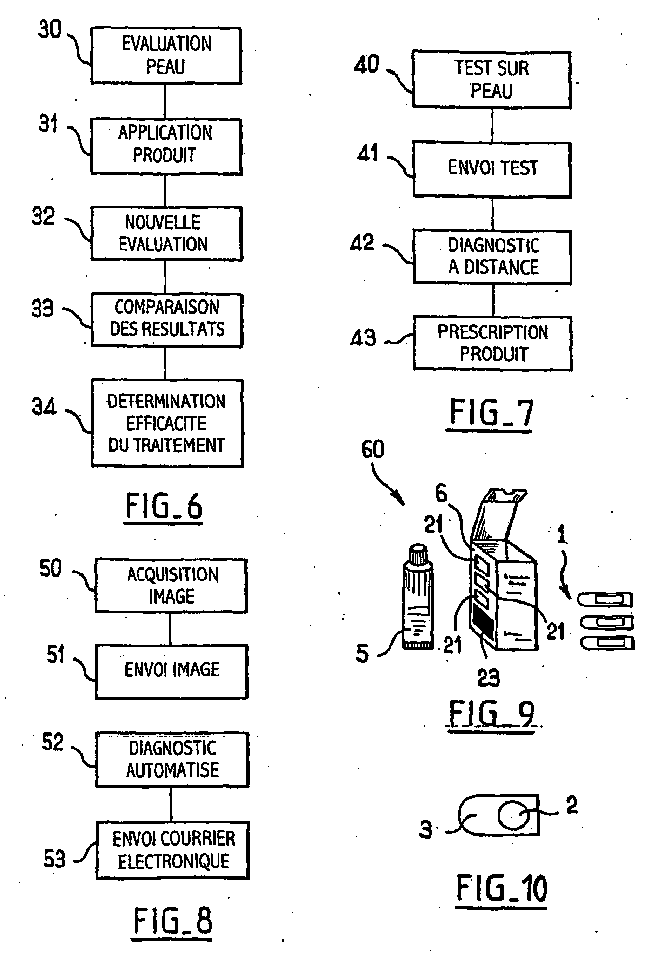

La figure 6 résume différentes étapes d'un procédé permettant de déterminer l'efficacité d'un traitement.

Dans ce procédé, on commence à l'étape 30 par effectuer une première évaluation de la peau, au moyen par exemple du procédé décrit en référence à la figure 3.

On applique ensuite à l'étape 31 un produit, par exemple un produit antirides, ayant une action sur les rides ou ridules présentes dans la zone de test sur laquelle a été appliqué le support 1.

On procède, après une ou plusieurs applications du produit, à l'étape 32, à une nouvelle évaluation de la zone de test.

Cette nouvelle évaluation est effectuée de la même manière que la première, avec un nouveau support 1, au moyen par exemple du procédé de la figure 3.

On procède ensuite à l'étape 33 à la comparaison des résultats des différentes évaluations afin de déterminer à l'étape 34 l'efficacité du traitement.

Le procédé de la figure 6 peut être mis en oeuvre par le consommateur lui-même ou par un professionnel dans un centre spécialisé ou sur un lieu de vente, par exemple.

L'analyse de l'image formée sur le support peut être effectuée à distance, par exemple de la manière illustrée à la figure 7.

Dans ce procédé, on commence par appliquer à l'étape 40 le support adhésif 1 sur une zone de test et l'on envoie à l'étape 41 ce support adhésif à un centre de diagnostic, lequel établit à distance, à l'étape 42, un diagnostic. La personne ayant subi le test peut recevoir à l'étape 43 le résultat de l'analyse, accompagné par la prescription d'un produit antirides par exemple.

Le support 1 peut être envoyé tel quel au centre de diagnostic, après application sur la peau.

On peut également procéder, comme illustré à la figure 8, à l'acquisition, dans une première étape 50, de l'image formée sur le support 1 au moyen d'une caméra ou d'un scanner, puis envoyer cette image sous la forme d'un fichier à l'étape 51 à un centre de diagnostic, en se connectant par exemple à un site Internet. Le diagnostic peut être effectué de manière automatique à l'étape 52, par comparaison automatique des images au moyen d'un moteur de reconnaissance de formes par exemple, puis le résultat de l'évaluation est envoyé à l'étape 53 à la personne ayant effectué le test, par un courrier conventionnel ou par un courrier électronique.

Le serveur Internet auquel les images sont envoyées peut être agencé pour mémoriser toutes les images reçues afin d'afficher par exemple par la suite ces images simultanément ou successivement, les comparer et déterminer l'efficacité d'un traitement ou de décider du besoin d'un traitement, par exemple.

Un ou plusieurs supports peuvent être commercialisés avec un produit antirides 5 et son emballage 6 sous la forme d'un kit.

Dans ce cas, l'emballage 6 comporte avantageusement un ensemble d'images de référence 21 formant un atlas d'auto-évaluation, ainsi qu'une zone sombre 23 facilitant l'observation de l'image formée sur le support adhésif 1.

Bien entendu, l'invention n'est pas limitée aux exemples qui viennent d'être donnés.

On peut notamment réaliser le support 1 sous de multiples formes, avec une surface adhésive ayant un contour circulaire, comme illustré à la figure 10, et le support peut être non transparent, par exemple de couleur foncée, afin d'éviter d'avoir à le placer devant une zone sombre.

## Revendications

1. Procédé d'évaluation du degré de vieillissement de la peau, comportant les étapes suivantes :
- appliquer un support (1) comportant une surface adhésive (2) sur une zone de test (T) de la peau,
- retirer le support,
- évaluer l'image formée sur la surface adhésive (2) du support (1) en la comparant avec des images de références d'un atlas présentant des motifs, notamment des lignes ou des points, et correspondant à divers degrés de vieillissement de la peau, l'image formée sur la surface adhésive du support résultant de la modification d'un aspect de la surface adhésive essentiellement aux endroits où la surface adhésive a été en contact effectif avec la peau, ladite surface adhésive n'ayant pas été en contact sensible avec les rides ou les ridules.

2. Procédé selon la revendication précédente, **caractérisé par le fait que** le support (1) est transparent.

3. Procédé selon la revendication précédente, **caractérisé par le fait que** l'examen du support s'effectue en le disposant devant un fond (4 ; 23) de couleur foncée.

4. Procédé selon la revendication 3, **caractérisé par le fait que** le support (1) est déposé sur le fond (4 ; 23) de couleur foncée sans le faire adhérer à ce dernier.

5. Procédé selon l'une quelconque des revendications précédentes, **caractérisé par le fait que** le support (1) comporte une languette de préhension (3) débordant d'un côté au moins de la surface adhésive (2).

6. Procédé selon l'une quelconque des revendications précédentes, **caractérisé par le fait que** ladite zone de test (T) est choisie parmi les régions suivantes :
- patte d'oie,
- front,
- coin de la bouche.

7. Procédé selon l'une quelconque des revendications précédentes, **caractérisé par le fait que** les images de référence (21) sont imprimées.

8. Procédé selon l'une quelconque des revendications 1 à 6, **caractérisé par le fait que** les images de référence (21) sont affichées à l'écran (E) d'un ordinateur.

9. Procédé selon l'une des revendications 1 à 8, **caractérisé par le fait que** la comparaison entre l'image formée sur la surface adhésive (2) et les images de référence (21) est effectuée à l'oeil nu.

10. Procédé selon l'une des revendications 1 à 8, **caractérisé par le fait que** la comparaison entre l'image formée sur la surface adhésive (2) et les images de référence (21) est effectuée de manière automatisée.

11. Procédé selon l'une quelconque des revendications précédentes, **caractérisé par le fait que** l'image formée sur la surface adhésive (2) est analysée à distance.

12. Procédé selon la revendication précédente, **caractérisé par le fait que** l'image formée sur la surface adhésive (2) est numérisée avant d'être analysée à distance.

13. Procédé selon la revendication 12, **caractérisé par le fait qu'**un traitement de l'image formée sur la surface adhésive (2) est effectué en vue de déterminer des paramètres caractéristiques de la zone de test.

14. Procédé selon l'une quelconque des revendications précédentes, **caractérisé par le fait que** l'on enregistre les images formées sur différents supports (1) appliqués successivement sur la zone de test (T).

15. Procédé selon la revendication précédente, **caractérisé par le fait que** ces images enregistrées sont comparées, notamment afin de mettre en évidence l'effet d'un traitement ou le besoin d'un traitement.

16. Procédé selon la revendication 14 ou 15, **caractérisé par le fait que** les images enregistrées sont affichées simultanément pour permettre à une personne de percevoir les effets d'un traitement ou de prendre conscience du besoin d'un traitement.

17. Kit comportant un produit (5) antirides, au moins un support (1) comportant une surface adhésive (2) destinée à être appliquée sur une zone de test (T) de la peau et un atlas (20) permettant d'évaluer le degré de vieillissement de la peau, l'atlas comportant une pluralité d'images de référence (21) représentative chacune de l'image formée sur un support (1) comportant une surface adhésive (2) après application sur une zone de test de la peau, ces images présentant des motifs et correspondant à différents degrés de vieillissement de la peau ;
l'atlas permettant par comparaison avec l'image formée sur la surface adhésive (2) du support, d'évaluer le degré de vieillissement de la peau.

## Claims

1. A method of evaluating the degree of aging of the skin, the method comprising the following steps:
- applying a medium (1) having an adhesive surface (2) onto a test zone (T) of the skin;
- removing the medium; and
- evaluating the image formed on the adhesive surface (2) of the medium (1), by comparison with reference images of an atlas presenting patterns, in particular lines or points, said image resulting from the appearance of the adhesive surface being modified essentially at locations where the adhesive surface has made actual contact with the skin, said adhesive not making significant contact with wrinkles or fine lines.

2. A method according to the preceding claim, **characterized by** the fact that the medium (1) is transparent.

3. A method according to the preceding claim, **characterized by** the fact that the medium is examined by being placed in front of a background (4; 23) of dark color.

4. A method according to claim 3, **characterized by** the fact that the medium (1) is placed on the background (4; 23) of dark color without being made to stick thereto.

5. A method according to any preceding claim, **characterized by** the fact that the medium (1) has a tongue (3) for handling purposes projecting from at least one side of the adhesive surface (2).

6. A method according to any preceding claim, **characterized by** the fact that said test zone (T) is selected from the following regions:
- the crow's foot;
- the forehead;
- the corner of the mouth.

7. A method according to any preceding claim, **characterized by** the fact that the reference images (21) are printed.

8. A method according to any one of claims 1 to 6, **characterized by** the fact that the reference images (21) are displayed on the screen (E) of a computer.

9. A method according to any one of claims 1 to 8, **characterized by** the fact that the image formed on the adhesive surface (2) is compared with the reference images (21) by the naked eye.

10. A method according to any one of claims 1 to 8, **characterized by** the fact that the image formed on the adhesive surface (2) is compared with the reference images (21) in automated manner.

11. A method according to any preceding claim, **characterized by** the fact that the image formed on the adhesive surface (2) is analyzed remotely.

12. A method according to the preceding claim, **characterized by** the fact that the image formed on the adhesive surface (2) is digitized prior to being analyzed remotely.

13. A method according to claim 12, **characterized by** the fact that the image formed on the adhesive surface (2) is treated in order to determine parameters that are characteristic of the test zone.

14. A method according to any preceding claim, **characterized by** the fact that the images formed on different media (1) applied in succession to the test zone (T) are stored.

15. A method according to the preceding claim, **characterized by** the fact that said stored images are compared, in particular for the purpose of showing up the effect of treatment or the need for treatment.

16. A method according to claim 14 or claim 15, **characterized by** the fact that the stored images are displayed simultaneously to enable a person to see the effects of treatment or to become aware of the need for treatment.

17. A kit comprising an antiwrinkle product, at least one medium (1) having an adhesive surface (2) for applying to a test zone (T) of the skin, and an atlas (20) for evaluating the degree of aging of the skin, the atlas comprising a plurality of reference images (21) each representative of an image formed on a medium (1) having an adhesive surface (2) that has been applied to the test zone of the skin, said images presenting patterns, corresponding to different degrees of aging of the skin ; the atlas enabling, by comparison with the image formed on the adhesive surface (2) of the medium, to evaluate the degree of aging of the skin.

## Patentansprüche

1. Verfahren zur Bewertung der Alterung der Haut, umfassend die folgenden Schritte:
- einen Träger (1), der eine klebende Fläche (2) umfasst, auf eine Testzone (T) der Haut auflegen,
- den Träger abziehen,
- das auf der klebenden Fläche (2) des Trägers (1) gebildete Bild bewerten, indem man es mit Bezugsbildern eines Atlas vergleicht, die Muster aufweisen, insbesondere Linien oder Punkte und die verschiedenen Graden der Alterung der Haut entsprechen, wobei das auf der klebenden Fläche des Trägers gebildete Bild sich aus der Änderung eines Aussehens der klebenden Fläche im Wesentlichen an den Stellen ergibt, an denen die klebende Fläche mit der Haut in tatsächlichem Kontakt war, wobei die klebende Fläche mit den Falten oder den Runzeln nicht in wesentlichem Kontakt war.

2. Verfahren nach dem vorhergehenden Anspruch, **dadurch gekennzeichnet, dass** der Träger (1) transparent ist.

3. Verfahren nach dem vorhergehenden Anspruch, **dadurch gekennzeichnet, dass** die Prüfung des Trägers stattfindet, indem er vor einem Hintergrund (4; 23) von dunkler Farbe angeordnet wird.

4. Verfahren nach Anspruch 3, **dadurch gekennzeichnet, dass** der Träger (1) auf dem Hintergrund (4; 23) von dunkler Farbe angeordnet wird, ohne ihn an diesem haften zu lassen.

5. Verfahren nach einem der vorhergehenden Ansprüche, **dadurch gekennzeichnet, dass** der Träger (1) eine Greifzunge (3) umfasst, die an mindestens einer Seite der klebenden Fläche (2) vorsteht.

6. Verfahren nach einem der vorhergehenden Ansprüche, **dadurch gekennzeichnet, dass** die Testzone (T) aus den folgenden Bereichen ausgewählt ist:
- Krähenfuß,
- Stirn,
- Mundwinkel.

7. Verfahren nach irgendeinem der vorhergehenden Ansprüche, **dadurch gekennzeichnet, dass** die Bezugsbilder (21) gedruckt sind.

8. Verfahren nach irgendeinem der Ansprüche 1 bis 6, **dadurch gekennzeichnet, dass** die Bezugsbilder (21) auf dem Schirm (E) eines Rechners angezeigt werden.

9. Verfahren nach einem der Ansprüche 1 bis 8, **dadurch gekennzeichnet, dass** der Vergleich zwischen dem auf der klebenden Fläche (2) gebildeten Bild und den Bezugsbildern (21) mit bloßem Auge durchgeführt wird.

10. Verfahren nach einem der Ansprüche 1 bis 8, **dadurch gekennzeichnet, dass** der Vergleich zwischen dem auf der klebenden Fläche (2) gebildeten Bild und den Bezugsbildern (21) automatisiert durchgeführt wird.

11. Verfahren nach einem der vorhergehenden Ansprüche, **dadurch gekennzeichnet, dass** das auf der klebenden Fläche (2) gebildete Bild femanalysiert wird.

12. Verfahren nach dem vorhergehenden Anspruch, **dadurch gekennzeichnet, dass** das auf der klebenden Fläche (2) gebildete Bild vor der Femanalyse digitalisiert wird.

13. Verfahren nach Anspruch 12, **dadurch gekennzeichnet, dass** eine Behandlung des auf der klebenden Fläche (2) gebildeten Bilds vorgenommen wird, um charakteristische Parameter der Testzone zu bestimmen.

14. Verfahren nach einem der vorhergehenden Ansprüche, **dadurch gekennzeichnet, dass** man die Bilder registriert, die auf verschiedenen, nacheinander auf die Testzone (T) aufgelegten Trägem (1) gebildet wurden.

15. Verfahren nach dem vorhergehenden Anspruch, **dadurch gekennzeichnet, dass** diese registrierten Bilder verglichen werden, um insbesondere die Wirkung einer Behandlung oder die Notwendigkeit einer Behandlung nachzuweisen.

16. Verfahren nach Anspruch 14 oder 15, **dadurch gekennzeichnet, dass** die registrierten Bilder gleichzeitig angezeigt werden, um einer Person zu gestatten, die Wirkungen einer Behandlung wahrzunehmen oder die Notwendigkeit einer Behandlung zu erkennen.

17. Set, umfassend ein Antifaltenprodukt (5), mindestens einen Träger (1), der eine klebende Fläche (2) umfasst, die dazu bestimmt ist, auf eine Testzone (T) der Haut aufgelegt zu werden, und einen Atlas (20), der es gestattet, den Grad der Alterung der Haut zu bewerten, wobei der Atlas eine Vielzahl von Bezugsbildern (21) umfasst, die jeweils das Bild darstellen, das auf einem eine klebende Fläche (2) aufweisenden Träger (1) nach Auflegen auf eine Testzone der Haut gebildet wurde, wobei diese Bilder Muster aufweisen und beispielsweise verschiedenen Graden der Alterung der Haut entsprechen;
wobei der Atlas durch Vergleich mit dem auf der klebenden Fläche (2) des Trägers gebildeten Bild gestattet, den Grad der Alterung der Haut zu bewerten.
